# EUROPEAN PATENT APPLICATION

(11) **EP 0 602 297 A1**
(43) Date of publication of application: **22.06.1994**
(21) Application number: 92830570.5
(22) Date of filing: 15.10.1992
(51) Int. Cl.: C07K 15/20, A61K 37/12, A61K 7/00

(54) **Fish collection and elastin solutions**

(30) Priority: 16.10.1991 IT MI912736
(71) Applicant: ANGRA S.r.l., I-20135 Milano (IT)
(72) Inventor: Skrodzki, Mieczyslaw, I-20135 Milano (IT)
(74) Representative: Cicogna, Franco

(57) **Abstract**

The present invention relates to a particular product comprising sea-collagen with elastin, having high purity and transparency features, and which, accordingly, can be used in the pharmaceutic and cosmetic field.

The invention also pertains to a method for making the subject product.

## Description

### BACKGROUND OF THE INVENTION

The present invention relates to a sea-collagen solution, which is particularly suitable for use in the cosmetic and pharmaceutic fields.

The invention also pertains to a method for preparing the above mentioned sea-collagen.

As is known, a collagen rich source consists of animals and fish and, in particular, the skin thereof.

The fish skin, derived from the fish processing industry, was considered as a waste product.

The collagen, on the other hand, is prepared from the skin by using an organic acid, usualy citric acid, and the raw collagen material, before the collagen withdrawal step, must be subjected to a preliminary processing step.

Heretofore, the animal skin was subjected to a preliminary alkaline-hydrochloric acid processing and to neutralization, before being processed with the mentioned organic acid.

As known, the fish skin is very delicate, and, accordingly, the application of a preliminary chemical processing causes the fish skin to be decomposed and to be transferred to the collagen solution under the form of a very fine suspension.

Moreover, under the same preliminary processing conditions, pigments are present in the processing solution, which cause the solution to become turbid.

Other drawbacks of prior methods comprise a poor quality of the obtained product which has great solution impurities.

Moreover, the obtained solution also has a poor purity and transparency.

Because of the product purity requirement, and the requirements related to its colour, prior collagen solution obtained from fish skin can not be used in the pharmaceutic and cosmetic field.

A sea-collagen and elastin solution was not previously known.

### SUMMARY OF THE INVENTION

According to the present invention, the solution of sea-collagen with elastin is characterized in that it contains the soluble elastin with a nitrogen contents of 1700-2100 mg/cm³, the total contents of nitrogen being of 6400-6800 mg/cm³, and the pH of the solution being included within the limits from 3.2 to 3.4.

The made solution has a clear colour, i.e. a light green-blue colour, because of the presence of the elastin, the ash contents being less than 0.26%, and the hydroxyproline aminoacid amount being less than 3,500 mg/cm³.

The method for making the sea collagen-elastin solution provides for the use of the Gadus family fish skin, and the use of a pre liminary processing step on the fish skin, comprising the mechanical removal of the fleshy and fat substance of the hypodermic layer of connective tissue.

This method further comprises the step of introducing the fish skin, at the end of the pre liminary processing, into a drum mixing apparatus provided with inner blades, and having a volume which is about three times the processed raw material amount, which has been preliminary processed in the mixing apparatus.

In this latter, the fish skins are mixed with water, in a ratio of 1.1:1.0, for a time of about 20-40 minutes, with a speed of 50 rpm's.

Then, the suspension of guanine with flakes and pigments is removed from the mixing apparatus drum, in order to add a further water amount and subject the mixture to a further mixing operation.

This operation is repeated so as to provide at the outlet of the drum a clear and clean liquid material, whereas the fish skins, as partially dehydrated, are subjected to a disinfection step by means of 0.2% hydrogen peroxide.

The withdrawing process is performed with a water solution of an organic acid, preferably citric acid, by holding its concentration value within the limits of 2.5-2.7, whereas the sea collagen-elastin solution is brought to a pH of 3.2÷3.5.

Nypagin is preserved with an alcohol saturated solution, used in an amount 0.2% by weight with respect to the starting raw material.

The obtained product is left for five days at a temperature of about 292°K, and then, after this period, it is subjected to a filtering step.

The nypagin to be used is the A type, or the M type, or, preferably, a mixture of these two types, with a 1:1.5 ratio, or a mixture of the types A+M+P, with a 1:1:1 ratio.

As known, nypagin A comprises the ethyl ester of the p-hydroxybenzoic acid.

Nypagin M comprises a methylester of the p-hydroxybenzoic acid.

Nypagin P comprises a propylester of the p-hydroxybenzoic acid.

From searches it has been found that the separation of the guanine crystals, flakes and pigments, before the withdrawing process on the fish skins, is such as to greatly improve the use values of the collagen solution.

By preserving given working parameters, in the drum mixing apparatus the fish skin is damaged.

Because of this damaging, the flakes, guanine and pigments are mixed with water and then, under a suspension form, are conveyed to the mixing apparatus and then can be subjected to a subsequent processing step.

The extraction of the skin, devoid of the above mentioned substance, by an organic acid, while holding the value of the pH at 2.4-2.7, provides a sea collagen having high purity characteristics and which can found a broad use.

It has been moreover found that, during the process, a great amount of soluble elastin is transferred to the collagen solution.

Moreover, the end decanting and filtering process, performed at room temperature, for several days, allows to eliminate both the unbound preserving agent and the remaining suspension.

A great advantage of the present invention consists of the possibility of providing a high purity product (clearness and transparency) and the reduction of the preparing time to 20 hours.

The presence of great amounts of elastin improves the use values of the collagen solution, whereas the reduction to 3.2-3.4 of the pH value of the collagen-elastin solution, jointly to a contents of ash less than 0.26%, improves the use features of the product, which is very suitable to be used in the cosmetic field.

The method according to the present invention will be disclosed in a more detailed way hereinafter with reference to some examples thereof.

**EXAMPLE 1:** 100 Kg of fresh "blue" fish have been subjected to a mechanical processing method.

This mechanical processing method is performed for removing, by means of a sharpened tool, the fleshy and fat tissue, and in a cold water rinsing step.

The drained skin, having a weight of about 33 Kg, is introduced into a drum of 100 l of a mixing apparatus, and is covered by about 30 l water.

The skin is mixed for 30 minutes, at a speed of about 50 revolutions/minute.

The suspension is removed, and the skin is subjected again to a mixing processing step, for a period of 25 minutes.

This operation is repeated until at the outlet of the drum a clean water is obtained.

The fish skin, with the skin flakes removed, and upon removal of guanine and pigments, is subjected to a disinfection process with a bath of 100 l containing 0.2% hydrogen peroxide for about 5 minutes.

Upon removal from the bath, the fish skin is rinsed by cold water and pressed in a press, in order to remove therefrom the excess water, so as to leave a residue amount of water of about 60%.

The raw material, upon dehydrating in the above disclosed manner, is supplipd to a withdrawing apparatus and covered by about 400 l of a citric acid solution having a value of pH of 2.4 and left for 20 hours at a temperature of 293°K.

The extracted material is filtered, the pH brought to 3.2 and preserved with the nypagin mixture A+M in a 1:1.5 ratio, in an amount of 0.2% with respect to the amount of the used raw material.

The obtained product is left at a temperature of 292°K for five days.

Upon this decanting period, the solution is filtered off.

**EXAMPLE** II: 100 kg of frozen mintaj fish skin are subjected to a thawing step.

A mechanical processing analogous to that of the Example I is performed, and then the material is rinsed by cold water.

The drained fish skin, for a total of 66 kg, is introduced into the mentioned drum mixing apparatus, having a volume of 200 l and being covered by 60 l water.

This mixture is blended for about 40 minutes at a speed of 50 rpm's.

The suspension is removed from the drum and the fish skin is covered by other 60 l water and mixed for further 30 minutes.

This operation is repeated until the water at the outlet of the drum is clean.

Then, the steps of the example I are repeated, by holding the pH of the boric acid to a value corresponding to pH 2.6, whereas the pH of the collagen solution is brought to a value of 3.5.

Then, the same method as that of the Example I is repeated.

The invention has been disclosed only by way of an exemplary but not limitative embodiment of the subject method and product, comprising a solution of sea collagen and elastin, and which is particularly suitable to be used in the cosmetic and pharmaceutic field.

Thus, the invention will be susceptible to several modifications and variations all of which will come within the scope and spirit of the appended claims.

## Claims

1. A solution characterized in that said solution comprises sea collagen with elastin and further comprises 1,700-2,100 mg N/cm³ nitrogen and with a total contents of nitrogen of 6,400-6,800 mg N/cm³, the pH value of said solution being included within the limits of 3.2 - 3.4.

2. A solution according to Claim 1, characterized in that said solution comprises high purity sea collagen and elastin, and can be used in the cosmetic field.

3. A solution according to Claim 1, characterized in that said solution comprises high purity sea collagen and elastin and can be used in the pharmaceutic field.

4. A method for making a sea collagen-elastin solution, said method comprising the step of preliminarily processing fish skin, said step comprising a mechanical removal, from the hypodermic layer of the connective tissue, of a fleshy and fat substance, characterized in that said method further comprises the step of introducing said fish skin into a drum mixing apparatus provided with a plurality of inner blades, said mixing apparatus having a volume which is three time the amount of the raw material introduced into said mixing apparatus, said fish skin being mixed with water in a 1.1:1.0 ratio for 20-40 minutes, at a speed of about 50 revolutions per minute, and then removing, from the drum of said mixing apparatus, a suspension of guanine, flakes and pigments, and then covering this mixture with a further water amount and subjecting it to a further mixing step by repeating the above disclosed steps until the water at the outlet of the drum is clean.

5. A method for making a sea collagen-elastin solution, according to Claim 4, characterized in that said method further comprises the step of subjecting the dehydrated skin fish to a disinfection step by hydrogen peroxide at 0.2%, and to a withdrawing step performed with a water solution of an organic acid, preferably citric acid, by holding the concentration at a pH value within the limits of 2.5-2.7 and by bringing the obtained extract to a pH of 3.2-3.5 and then preserving it with an alcoholic saturated solution of nypagin, used in an amount of 0.2% by weight, with respect to the starting raw material, whereas the solution is left at a temperature of 292°K for about five days, to be then subjected to a filtering step.

6. A method according to Claim 5, characterized in that it comprises the further step of using nypagin A or nypagin M, or a mixture thereof in a 1:1.5 ratio.

7. A method according to Claim 5, characterized in that it comprises the further step of using a nypagin mixture A+M+P in a 1/1/1 ratio.
